# FASCICULE DE BREVET EUROPEEN

(11) **EP 1 570 091 B1**
(45) Date de publication et mention de la délivrance du brevet: **08.02.2012**
(21) Numéro de dépôt: 03813618.0
(22) Date de dépôt: 04.07.2003
(51) Int. Cl.: C12Q 1/68, G01N 27/414

(54) **PROCEDE DE DETECTION ELECTRONIQUE D'AU MOINS UNE INTERACTION SPECIFIQUE ENTRE DES MOLECULES SONDES ET DES BIOMOLECULES CIBLES.**
METHODE ZUR ELEKTRONISCHEN DETEKTION WENIGSTENS EINER SPEZIFISCHEN INTERAKTION ZWISCHEN EINEM PROBEN- UND EINEM ZIELMOLEKÜL
METHOD FOR ELECTRONICALLY DETECTING AT LEAST ONE SPECIFIC INTERACTION BETWEEN PROBE MOLECULES AND TARGET BIOMOLECULES

(30) Priorité: 11.12.2002 WO PCT/FR02/04283
(43) Date de publication de la demande: 07.09.2005
(73) Titulaire: CENTRE NATIONAL DE LA RECHERCHE SCIENTIFIQUE (CNRS), 75794 Paris Cedex 16 (FR)
(72) Inventeur: BOCKELMANN, Ulrich, F-75020 PARIS (FR); POUTHAS, François, F-14530 Luc Sur Mer (FR); GENTIL, Cédric, D-81667 München (DE)
(74) Mandataire: Jacquard, Philippe Jean-Luc
(86) Numéro de dépôt international: PCT/FR2003/002091
(87) Numéro de publication internationale: WO 2004/057027

(56) Documents cités:
- WO-A-00/75276
- WO-A-03/052097
- WO-A-03/054225
- US-A- 4 238 757
- SOUTEYRAND E ET AL: "DIRECT DETECTION OF THE HYBRIDIZATION OF SYNTHETIC HOMO-OLIGOMER DNA SEQUENCES BY FIELD EFFECT" JOURNAL OF PHYSICAL CHEMISTRY. B, MATERIALS, SURFACES, INTERFACES AND BIOPHYSICAL, WASHINGTON, DC, US, vol. 101, 1997, pages 2980-2985, XP001040796 ISSN: 1089-5647
- TSURUTA H. ET AL.: "Detection of the products of apolymerase chain reaction by an ELISa system based on an ion sensitive field effect transistor" JOURNAL OF IMMUNOLOGICAL METHODS, vol. 176, 1994, pages 45-52, XP009021947
- M. NAKAMURA AND M. YANE: "Online pH, pCO2, pO2 monitoring system using two-way cyclic pumping of lactate Ringer as a baseline solution" MEICAL & BIOLOGICAL ENGINEERING & COMPUTING, janvier 1987 (1987-01), pages 45-50, XP001156254
- KIESSLING V ET AL: "EXTRACELLULAR RESISTANCE IN CELL ADHESION MEASURED WITH A TRANSISTOR PROBE" LANGMUIR, ACS, WASHINGTON, DC, US, vol. 16, 2000, pages 3517-3521, XP001040795 ISSN: 0743-7463

## Description

La présente invention a pour objet un procédé de détection électronique d'au moins une interaction entre des molécules et des biomolécules cibles.

On connaît déjà un procédé de détection de l'hybridation de séquences d'ADN à l'aide d'un transistor à effet de champ, tel qu'il a été décrit dans l'article de E. SOUTEYRAND et collaborateurs intitulé « Direct Detection of the hybridization of synthetic Homo-Oligomer DNA Sequences by Field Effect » paru en 1997 dans le J. Phys. Chem. B1997, 101, pages 2980 à 2985. Un transistor de type ISFET (« Ion-Sensitive - Field Effect Transistor ») utilisable dans ce type d'application a été décrit dans l'article de Piet BERGVELD « Development, Operation and Application of the ISFET as a Tool for Electrophysiology paru dans IEEE Transactions on Biomedical Engineering volume BME-19 - n° 5 Sept 1972 pages 342 à 351. Des indications sur la fabrication de telles structures de transistors peuvent être trouvées dans l'article de V. KIESSLING et collaborateurs, intitulé « Extracellular Resistance in Cell Adhesion Measured with a Transistor Probe » paru dans Langmuir 2000 16, pages 3517 - 3521. Enfin un mode de préparation des surfaces a été décrite dans l'article de A. KUMAR et collaborateurs, intitulé « Silanized nucleid acids : a general platform for DNA immobilization » paru dans Nucleid Acid Research 2000, volume 28, n° 14, pages i à vi.

Deux procédés de fixation des molécules sondes sur la surface sont notamment utilisables dans le cadre de la présente invention. Le premier consiste en une synthèse directe sur solide, telle que décrite par exemple dans l'article de S.P.A. Fodor et Collaborateurs intitulé « Light-directed, spatially adressable parallel chemical synthesis » paru dans Science 251, pages 767 à 773 (1991). Le deuxième est la fixation des molécules à partir d'une dilution.

Pour obtenir une bonne détection des interactions entre des biomolécules, il convient de prendre en compte un certain nombre de paramètres :

### A- Sensibilité

Elle peut être très bonne (comparable à celle d'une détection par fluorescence) mais elle dépend de l'état des couches moléculaires sur les surfaces actives. Pour les capteurs du type transistor qui sont utilisés ici, la quantité nécessaire de molécules biologiques (sondes et cibles) est inversement proportionnelle à la surface du capteur. On a donc intérêt à miniaturiser les structures, mais il faut veiller au rapport signal/bruit.

### B- Dynamique

L'intervalle des différentes concentrations de molécule qu'on peut mesurer est étroit. A faible concentration, on peut être limité par des signaux parasites, (p.ex. on peut voir parfois déjà un signal induit par une goutte d'eau pure qui avait séché sur la surface). A forte concentration, on observe une saturation lorsque la charge effective s'approche de zéro.

### C- Spécificité

Par spécificité, on entend la capacité du système de distinguer entre deux types de molécules cibles différentes. Pour l'hybridation entre des molécules d'ADN par exemple, cette différence peut être une différence dans la séquence des paires de bases. Il s'agit d'optimiser les conditions (sel, température, durée, et éventuellement pH) de la réaction de reconnaissance intermoléculaire de façon à ce que l'interaction spécifique (p.ex. l'hybridation entre des séquences complémentaires) domine par rapport à des processus non-spécifiques (p.ex. l'adsorption, les interactions ioniques et hydrophobes non spécifiques). Ces conditions optimisées ne correspondent en général pas aux meilleures conditions pour une détection électronique. Par exemple, on utilise typiquement plus de sel pour une hybridation spécifique que pour une détection électronique. Un compromis est à trouver aussi pour la distance des molécules sondes par rapport à la surface. Une faible distance est favorable pour la détection électronique à cause de l'écrantage électrostatique mais souvent défavorable pour la spécificité. Si les molécules sondes sont trop proches de la surface en SiO₂, les interactions non spécifiques molécule/surface peuvent devenir dominantes. De plus, l'interaction spécifique entre les bio molécules sondes et cibles peut être gênée par une surface à proximité (problème d'accessibilité ou empêchement stérique).

Ces effets dépendent des couches moléculaires déposées sur la surface active du réseaux de transistor (et en particulier de leur état de charge).

Le Brevet US 4 238 575 (SCHENCK) propose une mesure de concentration qui consiste à mesurer la pente du courant de drain d'un seul transistor. Cette mesure ne permet pas d'éliminer le biais que constituent les modifications de l'état de surface causées par le changement de sel que ce procédé implique.

Dans la description ci-après, on a proposé une approche expérimentale permettant d'aboutir au résultat recherché. Cette approche a permis de mettre en évidence un point clé de la détection, qui constitue, l'idée de base de la présente invention, à savoir qu'il convenait de séparer la réaction de reconnaissance de l'étape de détection et effectuer une mesure différentielle.

La présente invention concerne ainsi un procédé de détection électronique d'au moins une interaction spécifique entre des molécules sondes fixées au moins à une zone active d'un capteur et des biomolécules cibles, caractérisé en ce que ledit capteur est constitué par un réseau de transistors à effet de champs (T₁, T₂, ...) dont chacun présente une région de source (5), une région de drain (D) ainsi qu'une région de porte qui constitue une zone active (3) sur laquelle ladite interaction spécifique doit être détectée, tel que défini dans la revendication 1 :

D'une manière générale, on considère comme molécule sonde toute molécule, notamment les biomolécules (ADN, ARN, protéines, etc...), ou bien l'ADN oligonucléotide synthétisé chimiquement, ou bien encore les acides peptides nucléiques ("PNA"), qui peut être greffée sur une dite surface active et qui peut être reconnue spécifiquement par une biomolécule cible.

Selon une première variante dite spatiale, la mesure différentielle de l'étape c) est effectuée entre deux groupes de molécules sondes du même type par exemple (ADN), ayant ou non les mêmes séquences, fixées sur des zones actives distinctes, l'un des groupes ayant été soumis à l'interaction de l'étape b), et l'autre pas.

Selon une deuxième variante dite spatiale, la mesure différentielle de l'étape c) est réalisée avec le tampon de mesure sur deux groupes de molécules sondes disposés sur des zones actives distinctes, ces deux groupes de molécules sondes, du même type, mais ayant ou non les mêmes séquences, et ayant été soumis à des interactions spécifiques différentes.

Les molécules sondes et/ou les biomolécules cibles sont par exemple des molécules d'ADN, d'ARN, de "PNA", de protéines, ou bien encore des petites molécules telles que les vitamines. Le tampon réactionnel et le tampon de mesure sont par exemple du KCI. La concentration en sel du tampon de mesure est par exemple supérieure à 0,002 mM et inférieure à 20 mM, et elle est par exemple supérieure à 0,005 mM et inférieure à 20 mM, et notamment comprise entre 0,005 Mm et 15 mM, ou bien encore entre 0,01 mM et 15 mM. La concentration du rampon réactionnel est par exemple comprise entre 20 mM et 1 M (concentration molaire).

Le procédé selon l'invention est compatible avec une détection classique d'interaction moléculaire par fluorescence.

Le procédé peut être caractérisé en ce que ladite mesure d'au moins un point de la caractéristique met en oeuvre l'application d'une tension donnée (U_{DS}) entre le drain et la source d'au moins un transistor, ainsi que l'application dans un premier cas d'une tension donnée (U_{GS}) entre la porte et la source dudit transistor ou, dans un deuxième cas d'un courant de drain (I_{D}) donné audit transistor. Dans le premier cas, la mesure du point caractéristique consiste en la mesure du courant de drain Id. Dans le deuxième cas, on mesure la tension U_{GS.}

Selon une variante, on réalise une circulation à travers au moins un microcanal fluidique d'au moins une solution qui constitue une référence ou qui contient des molécules cibles pour la mettre en contact avec au moins une zone active d'un transistor à effet de champ.

On peut par exemple mettre une solution de référence (par exemple tampon de sel) et une solution contenant des molécules cibles successivement en contact avec une ou plusieurs zones actives, ou bien (en parallèle) avec une ou plusieurs zones actives différentes. On peut par exemple mettre en contact deux solutions de molécules cibles avec une ou plusieurs zones actives différentes en parallèle.

D'autres caractéristiques et avantages de l'invention apparaîtront mieux à la lecture de la description ci-après, en liaison avec les dessins annexés dans lesquels :
- la figure 1 représente deux transistors à effet de champ d'une puce de détection comprenant une pluralité de tels transistors organisés suivant un réseau mono ou bidimensionnel de transistors ;
- la figure 2 représente en vue de dessus un détail d'une puce de détection et l'agencement des zones actives correspondant chacune à un transistor à effet de champ ;
- la figure 3 illustre les connexions électriques de drain des transmissions du réseau mono ou bidimensionnel.
- la figure 4 représente un dispositif de dépose de la solution sur des zones actives sélectionnées ;
- les figures 5a à 5c représentent les résultats d'expériences réalisées dans différentes conditions expérimentales ;
- les figures 6a et 6b montrent une détection électronique d'ADN ;
- les figures 7a et 7b illustrent la mise en oeuvre de canaux microfluidiques ,
- et les figures 8a à 8h et 9a à 9d illustrent deux exemples de mise en oeuvre de l'invention.

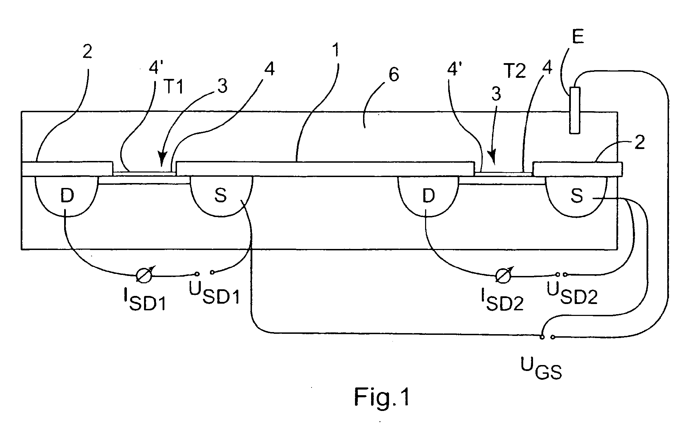

Les figures 1 à 3 illustrent un capteur présentant un réseau de transistors à effet de champ FET sur un substrat en silicium. Un transistor T₁ ou T₂ représenté en coupe à la figure 1 est pourvu d'une région de source S et d'une région de drain D qui présentent chacune un contact électrique et qui sont surmontées d'une couche isolante respectivement 1 et 2, par exemple un oxyde thermique de SiO₂. La région active 3 entre la source S et le drain D forme la région de porte G du transistor et présente une couche isolante 4 d'épaisseur réduite, par exemple une couche de SiO₂ thermique. Il est également possible de ne pas disposer d'oxyde sur cette région active. La surface active est alors délimitée par une portion 4' du substrat qui est mise à nu.

Des molécules sondes, par exemple des molécules d'ADN simple brin, sont fixées par un procédé connu sur au moins certaines des surfaces actives 4 ou 4'. Pour l'ADN, on utilise de préférence des transistors à effet de champ à canal n à appauvrissement (pour lesquels les porteurs de charge sont les électrons, plus mobiles d'où une augmentation de la sensibilité) avec une polarisation de porte négative (c'est-à-dire que l'électrolyte est polarisé négativement par rapport au semi-conducteur), l'ADN se chargeant négativement (pour un électrolyte de pH neutre).

L'application d'une tension source-drain U_{SD} entre la source S et le drain D (U_{SD1} pour T₁, et U_{SD2} pour T₂) et d'une tension porte-source U_{GS} entre l'électrolyte 6 et la source S (par exemple par une électrode E unique Ag/AgCl) induit un gaz bidimensionnel de porteurs de charges à l'interface Si/SiO₂, ou à l'interface Si/électrolyte de chaque transistor. Il en résulte un courant de drain I_{D} qui, pour chaque transistor, dépend de façon sensible de la charge à l'interface Sio₂/électrolyte ou Si/électrolyte. On appelle surface active cette interface qui fait face au canal entre la source S et le drain D.

Le courant I_{D} dépend de la fixation des molécules sondes, par exemple des molécules d'ADN sur la surface active 4 ou 4'.

On peut réaliser une détection à tension source-porte U_{SG} et à tension source-drain U_{SD} constants, en mesurant le courant de drain I_{Sd} ou bien encore, comme dans les exemples 1 et 2 ci-après, à courant de drain I_{Sd} et à tension source-drain U_{SD} constants, en mesurant la tension source-porte U_{SG}.

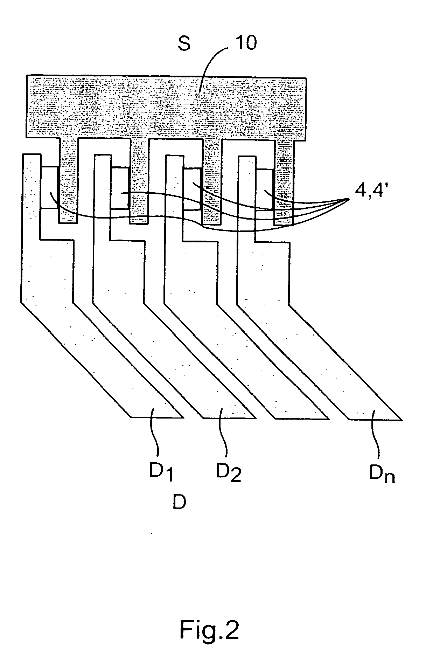

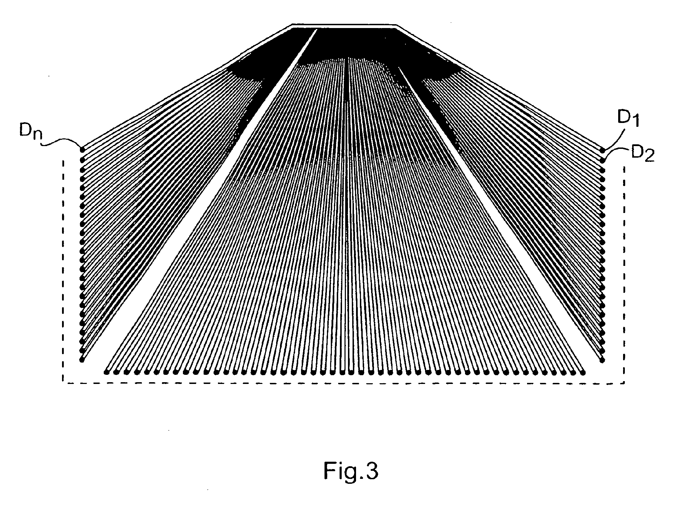

Comme le montrent les figures 2 et 3, n structures de type transistor à effet de champ sont intégrées dans un substrat de silicium recouvert d'un isolant (SiO₂ ou autre) et pourvu de connexions appropriées (métallisation ou de préférence régions conductrices dopées) par les prises de contact électrique de source 10 et de drain (D₁, ... Dₙ). A la différence d'une structure de transistor MOS standard, il n'y a pas d'électrode métallique de porte. Ceci correspond à la structure de type « ISFET » (« Ion Sensitive Field Effect Transistor »). On utilise de préférence un substrat de type SOI (silicium sur isolant) qui procure une sensibilité plus élevée.

Les différentes structures sont à faible distance latérale l'une de l'autre et leurs surfaces actives sont en contact avec la même solution de mesure. Une dimension latérale typique dans la micro-électronique actuelle est inférieure au µm. Dans la technologie des puces à ADN telle que mise en oeuvre dans la présente invention, la dimension latérale est de 5-10 µm pour une synthèse directe sur le solide et de 50-100 µm dans le cas d'une fixation des molécules à partir d'une dilution.

Dans la présente configuration de mesure parallèle, plusieurs plots avec différents types de molécules sondes immobilisées sont en contact avec la même solution notamment de mesure et au moins une structure de transistor se situe en dessous de chaque plot. La mise en oeuvre de plusieurs transistors par plot est possible au vu des dimensions mentionnées ci-dessus et permet une redondance dans la détection.

Une électrode E (Ag/AgCl, par exemple), est utilisée pour fixer le potentiel de la solution de mesure 6 (électrolyte) par rapport à la structure en silicium qu'elle recouvre et pour fixer le point de travail des capteurs (transistors). Le potentiel de l'électrolyte 6 peut dans certains cas être égal à zéro. La solution de mesure 6 qui baigne les capteurs contient des ions à une concentration qui donne une conductivité suffisante et qui ne donne pas lieu à un écrantage trop important au niveau des surfaces actives. Son pH est de préférence neutre.

La technique proposée permet de faciliter la détection à l'aide de différentes approches

### A- Caractériser les couches par mesures électroniques

La mesure électronique permet une caractérisation grossière des états (électrostatiques) des couches moléculaires déposées sur les transistors. On effectue des mesures électroniques entre les différentes étapes de préparation de surface. Chaque étape de traitement chimique ou de dépôt de molécule induit un décalage de la tension mesurée et les différences entre transistors reflètent une inhomogénéité éventuelle. L'ensemble de ces décalages donne alors une caractérisation du système avec de multiples couches déposées.

En d'autres termes, on peut ainsi vérifier l'état "électrochimique" du réseau de capteurs. On peut comparer à des valeurs de références mesurées pour des conditions qu'on avait auparavant optimisé et on peut surveiller le vieillissement des structures (par exemple la dégradation des réseaux par dépôts de charges et autres effets sur l'oxyde SiO₂). De plus, la comparaison de deux mesures électroniques séparés par un petit intervalle de temps (et éventuellement un rinçage intermédiaire) permet de vérifier l'absence de dérive importante dans le signal électronique. Ces points sont importants car la réponse des transistors à effet de champ FET à l'immobilisation de molécules sur la surface active dépend de l'ensemble des couches sur la surface active. La procédure aide à obtenir de façon reproductible une sensibilité adaptée (à la quantité de molécules qu'on souhaite détecter) et une spécificité suffisante.

Une fois que le réseau de capteur est connu, les mesures peuvent être répétées sans procéder à de nouvelles vérifications.

### B- Séparer la réaction de reconnaissance de l'étape de détection.

Le dépôt des sondes, la réaction de reconnaissance et la mesure électronique peuvent être effectués dans différentes tampons. Ceci permet d'optimiser ces étapes de façon largement indépendante. On montre notamment ci-après l'exemple d'une hybridation avec une concentration en sel de 50mM couplée à une détection électronique avec une concentration en sel de 0,01 mM.

### C- Mesure différentielle

L'approche différentielle, sujet de la demande PCT/FR02/04283 dont la présente demande revendique la priorité, est un élément très important ici. Par rapport à la caractérisation des couches par mesure électronique, elle permet de détecter des inhomogénéités. Par rapport à la séparation de la réaction de reconnaissance de l'étape de détection, elle semble être même indispensable, car le décalage de la valeur de la tension U_{GS} induit par un changement de tampon montre souvent une variation plus importante que le signal correspondant à l'interaction spécifique qu'on souhaite détecter.

### D- Structures actives

L'utilisation des structures à effet de champ FET permet une taille caractéristique de l'ordre de 1 micron. L'approche par mesure en courant alternatif AC est utilisée par Fritz et al. (Proceedings Nat. Acad. Sci., USA, Vol. 99, p. 14142 (2002)). L'approche alternative d'une structure passive SᵢO₂/Sᵢ sans drain ni source (dite "spectroscopie d'impédance") nécessite une surface d'environ 50 microns par 50 microns en raison de la capacité parasite due aux connexions [(voir WIEGAND et al. Review of Scientific Instruments n° 71, 2309 (2000)]. On pourrait contourner cette difficulté par un traitement du signal alternatif dans la puce même, à proximité du capteur.

Mais même dans ce cas, l'électronique de mesure serait plus compliqué que celle mise en oeuvre dans le cadre de la présente invention pour la mesure en courant continu DC basée sur les transistors à effet de champ FET. Les structures actives (FET) sont donc plus adaptées à une miniaturisation. En mettent en oeuvre des surfaces actives de 2 microns par 20 microns, on n' est pas limité par le bruit des transistors FET.

Le procédé de détection des reconnaissances moléculaires peut être basée sur une approche par comparaison, notamment différentielle. La mesure est par exemple réalisée en utilisant plusieurs structures de transistor. La mesure peut être différentielle par rapport aux différents types de molécules greffées et inclure éventuellement plusieurs transistors par type de molécule. Il permet de comparer des signaux avant/après la réaction d'interaction qui révèle la reconnaissance moléculaire (et/ou l'évolution pendant cette réaction). On notera que la mesure de référence peut être réalisée dans le tampon de mesure ayant la deuxième concentration, mais également dans un autre tampon, par exemple dans ledit tampon réactionnel ayant une première concentration en sel.

Le procédé selon l'invention permet de contourner les difficultés associées à la sensibilité d'un capteur individuel au pH et à la force ionique et celles associées à une variabilité d'un transistor individuel à l'autre (ceci inclut la structure de transistor et la qualité de la fixation des sondes).

Un procédé selon un mode de réalisation met en oeuvre les étapes suivantes :
a) traitements homogènes de toute la surface isolante pour préparer la fixation des molécules sondes ;
b) greffage local de différents types de molécules sondes sur au moins certaines des surfaces actives individuelles ;
c) éventuellement rinçages homogènes ;
d) mesure électronique : ajouter l'électrolyte de mesure, plonger l'électrode et mesurer les transistors (par exemple un ou plusieurs points de la caractéristique I_{D} en fonction de U_{SD} et de U_{SG}), et comparer les résultats obtenus selon les transistors ;
e) éventuellement rinçages homogènes ;
f) ajout de la solution de molécules cibles en présence d'électrolyte et réaction de reconnaissance ;
g) éventuellement rinçages homogènes ;
h) mesure électronique, comme (d).

Certains transistors qui n'ont pas été mis en présence de molécules sondes (ou bien un seul transistor) peuvent servir de témoin. On mesure leurs caractéristiques après ajout de l'électrolyte de mesure qui par exemple, baigne l'ensemble des transistors.

Le greffage des molécules sondes est réalisé par dépôt de micro-gouttelettes de diamètre environ 100 µ sur les surfaces actives des transistors à l'aide de micro-plumes métalliques qui sont disponibles commercialement, ou bien un système de microdéposition commercial (par exemple Nanoplotter NP1 de la Société Ge Sim).

Comme le montre la figure 3, le réseau de n transistors (par exemple n = 96 transistors) présente n connexions de drain D₁, D₂ ... Dₙ et 2 connexions (non représentées) équivalentes à la source commune. Les résistances série R_{c} associées à ces connexions ont des valeurs qui dépendent de l'index 1 ...n du drain.

Les valeurs de ces résistances R_{c}, réalisées par exemple par dopage du silicium, ne sont pas négligeables, mais elles peuvent être corrigées.

A cet effet les résistances Rc de connexion de drain sont par exemple calculées à partir des longueurs et des sections géométriques des lignes dopées dont on connaît la résistivité. Le calcul est comparé à une mesure de la résistance en fonction de l'index du drain en appliquant une tension continue (par exemple U_{SD} = 0,1V et U_{SG} = 2V).

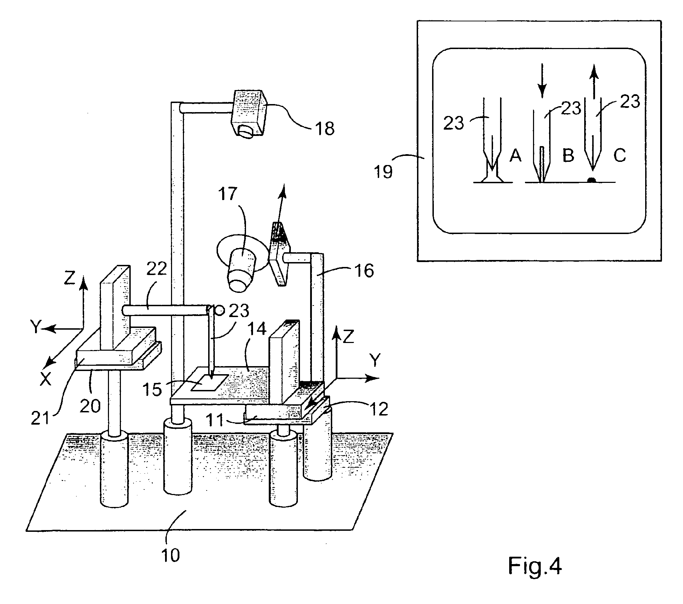

Une installation telle que celle représentée à la figure 4 peut être utilisée pour la mise en oeuvre du procédé : sur une table 10, est disposée une platine 12 incorporant un dispositif de commande à micro-contrôleur pour une table 11 assurant un déplacement selon trois directions perpendiculaires X, Y, Z. Une puce 15 incorporant le réseau de n transistors est disposée sur un support 14. Une autre platine 20 comportant une table 21 assurant un déplacement selon les trois directions X, Y, et Z est mise en oeuvre pour déplacer un bras 22 portant une micro-plume ou une pipette 23 pour assurer le dépôt des micro-gouttelettes sur au moins certains des n transistors. Un objectif 17 et/ou une caméra couplée à un écran 19 permettent d'observer le dépôt des micro-gouttelettes et de contrôler les opérations.

On effectue des mesures de courant de drain I_{D} avec par exemple U_{SG} = 1V et U_{SD} = 0,9V et un électrolyte de pH neutre déposé qui est constitué de KCI à une teneur de 0,1 millimole par litre. Les transistors (canal p à accumulation) ayant leurs sources interconnectées, la tension de source ou la tension de porte peut servir de référence de tension (par exemple la tension de masse).

Avant ces mesures, on effectue un traitement global de la surface de la structure Si/SiO₂ par incubation 1-2 minutes dans de l'acide sulfochromique et rinçage sous un courant d'eau déionisée puis incubation 3 à 5 minutes dans une solution de NaOH (60 µl NaOH 16N, 420µl d'éthanol et 220 µl d'eau), et enfin rinçage sous un courant d'eau déionisée.

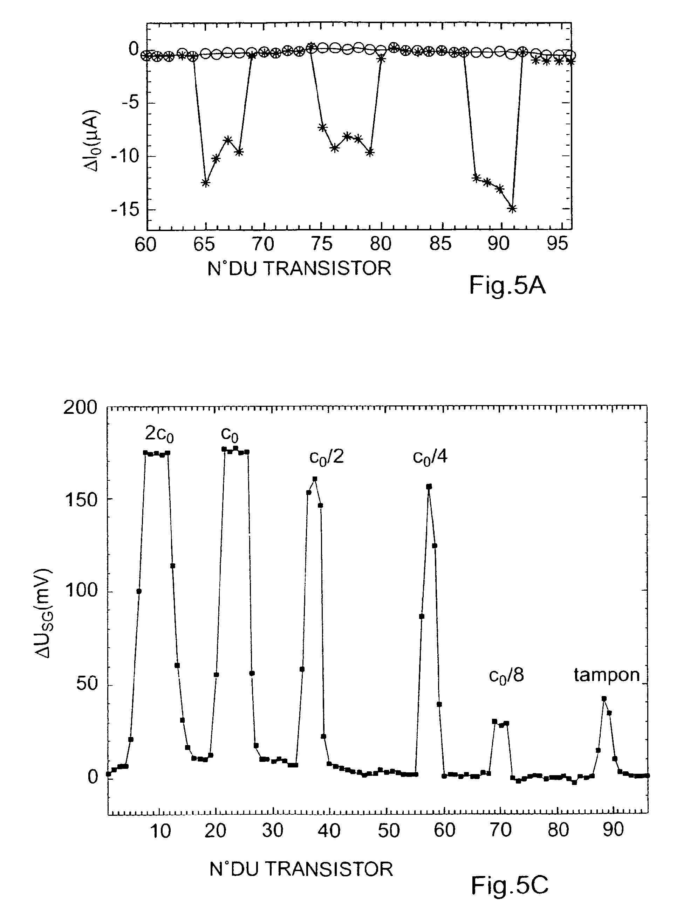

La différence entre deux mesures effectuées avant dépôt local mais avant et après un rinçage à l'eau est présentée en petits carrés à la figure 5a. Les croix représentent la différence entre une mesure effectuée après dépôt local de deux solutions différentes et une mesure faite avant le dépôt (la mesure faite avant le rinçage à l'eau).

Avec une pointe commerciale 23 (Telechem SMP3B) montée sur le dispositif 22 présenté à la figure 4, on dépose une solution de poly-L-lysine sur les transistors 64-69, les transistors 74-79, et les transistors 87-91.

Solution : Poly-L-lysine (0,01% poids/volume « w/v » final (P8920, Sigma)) dans un tampon PBS 0,1 X à pH 7.

Après les dépôts locaux, l'échantillon est séché 15 minutes en atmosphère humide et ensuite 5 minutes à 50°C.

La poly-L-lysine est positive dans l'électrolyte de mesure (pH neutre) à cause des groupements amines ionisés. La diminution du courant observé sur les dépôts de poly-L-lysine est compatible avec l'adsorption d'une charge positive sur la surface.

On mesure la différence de potentiel de surface ΔU_{SG} correspondant à la mesure avant/après dépôt. Pour déterminer ΔU_{SG}. on mesure la caractéristique bidimensionnelle par exemple I_{D}(U_{SG}, U_{SD}) et on détermine les caractéristiques intrinsèques des 96 transistors en corrigeant numériquement les caractéristiques mesurées en fonction des résistances R_{c} des lignes de drain en série. La modification de l'état de l'interface SiO₂ induit un changement de la caractéristique intrinsèque qui correspond à un décalage ΔU_{SG} à U_{SD} et courant de drain I_{D} constants. Ce décalage permet d'obtenir directement une mesure indépendante du point de travail du transistor, contrairement au changement de courant ΔI_{D} présenté dans la figure 5a. La valeur ΔU_{SG} permet en première approximation de quantifier le changement de l'interface SᵢO₂/liquide induit par le dépôt local. Selon une variante, on fait varier U_{SG} de façon à garder I_{D} constant.

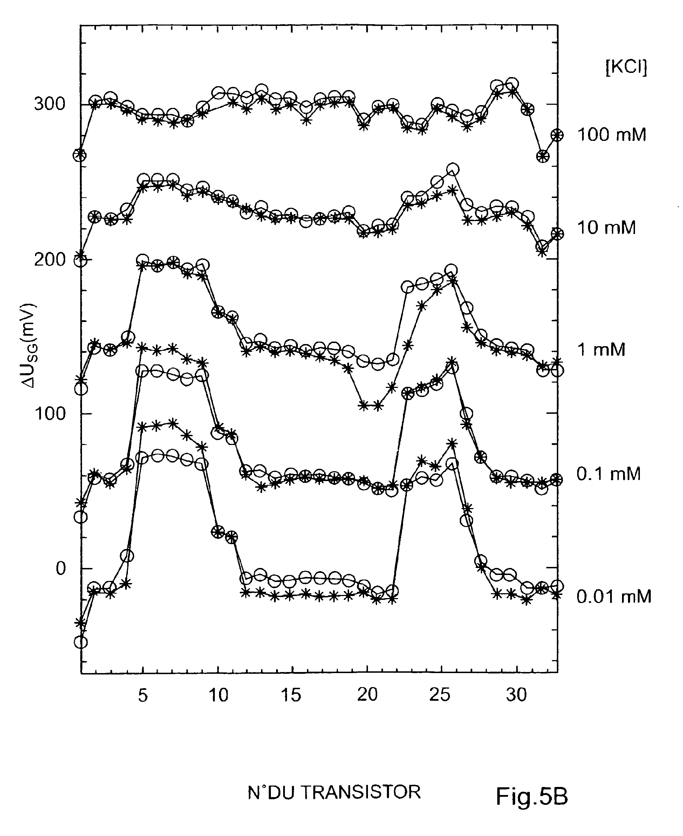

Les figures 5a à 5c montrent des mesures différentielles réalisées avant et après dépôt de poly-L-lysine (figure 5a), réalisées en fonction de la concentration en KCI (figure 5b), et réalisées en fonction de la concentration en poly-L-lysine déposée.

A la figure 5a, les variations ΔI_{D} du courant de drain Id sont représentées en ordonnée pour chacun des transistors 60 à 96 identifiés en abscisse (U_{SG} = 1 V, U_{SD} = 0,9 V et électrolyte KCI à 0,1 mM). Les différences ΔI_{D} entre deux mesures réalisées avant un dépôt local mais séparées par un rinçage à l'eau sont représentées par des cercles. Les différences ΔI_{D} correspondant à des mesures effectuées avant et après un dépôt local de poly-L-lysine sont représentées par des étoiles. Après le dépôt local on laisse l'échantillon 15 minutes à température ambiante en milieu humide, avant de le sécher à 50°C pendant 5 minutes. La dilution C_{O} de la poly-L-lysine est de 0,01 % poids/volume "W/V" final (P8920, Sigma) dans un tampon PBS 0,1 x à pH 7.

A la figure 5b, les différences ΔU_{SG} de la tension de source-grille U_{SG} sont mesurées sur une partie des transistors d'un réseau de 62 transistors FET avec U_{SD} = 1,2 V et I_{D} = 50 µA. Les différences entre une mesure de référence (effectuée avant dépôt local et avec une concentration en KCI de 0,01 mM) et de deux séries de mesures (effectués après dépôt local de poly-L-lysine et avec différentes concentrations en KCI) sont représentées par des cercles et des étoiles. Ici, un dépôt local de poly-L-lysine a été effectué dans deux zones distinctes avec la même dilution C_{O} que dans le cas de la figure 5a. Lors de chacune des deux séries de mesure, on fait varier la concentration de KCI dans le tampon de mesure entre 0,01 mM et 100 mM, en passant par les valeurs 0,1 mM, 1 mM et 10 mM . Entre les deux séries de mesure, on rince la surface à l'eau. On constate une sensibilité appréciable de la détection de la poly-L-lysine pour des concentrations de KCI entre 0,01 mM et 1 mM, et la hauteur des pics diminue progressivement au-delà de ces valeurs.

La figure 5c montre les variations ΔU_{SG} de la tension U_{SG} en fonction de la concentration en polymère déposé (poly-L-lysine), à savoir 2C_{O}, C_{O}, C_{O}/2, C_{O}/4, C_{O}/8 dans un tampon 0,1 x PBS pH 7, C_{O} ayant la valeur indiquée pour les mesures de la figure 5a. Les conditions de mesure sont les suivantes : U_{SD} = 1V, I_{D} = 100 µA, et une concentration de 0,01 mM pour KCI. Ces mesures montrent qu'il n'y a pas avantage dans les conditions expérimentales choisies d'augmenter la concentration au-delà de C_{O}.

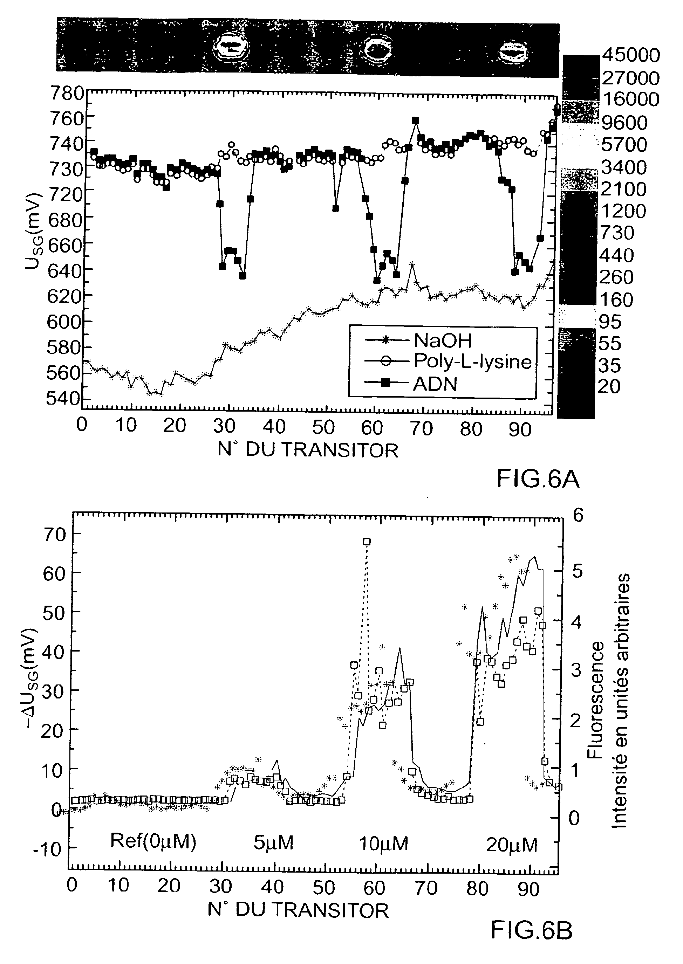

Les figures 6a et 6b montrent la détection électronique d'ADN. Les tensions U_{SG} et les variations ΔU_{SG} de la tension U_{SG} correspondent à un point de fonctionnement U_{SD} = 1V, I_{D} = 100 µA, et à une concentration 0,01 mM en KCl. Elles sont obtenues à partir de la caractéristique I_{D}(U_{SG}. U_{SD}) et sont reportées sur les courbes avec en abscisse le numéro des transistors FET (1 à 96).

Les étoiles représentent la mesure après traitement de surface initial à la soude. Les cercles représentent la mesure après incubation de poly-L-lysine sur tout le réseau. Pour permettre une immobilisation de l'ADN, on incube le réseau de transistors FET pendant 30 minutes dans une dilution de poly-L-lysine (concentration Co). Ensuite, sans effectuer de séchage préalable, on rince à l'eau et on sèche ensuite à l'air. L'incubation conduit à des décalages de la tension U_{SG} d'une valeur de 97 ± 50 mV (statistique sur 67 surfaces préparées) qui réduisent les variations entre transistors dans le signal électronique. Ces décalages sont compatibles à ceux observés avec les valeurs mesurées en relation avec la figure 5c sur des dépôts locaux à la même concentration. Les carrés représentent les mesures après dépôt local d'oligonucléotides (5' Cy-5 modifiés 20 mer, concentration 50 µM dans de l'eau désionisée) autour des transistors n° 30, 60 et 90. En niveau de gris et au dessus de la figure 6a, est représentée l'image en microfluorescence des trois points d'ADN précités.

La figure 6b montre la détection électronique et par fluorescence d'oligonucléotides Cy5 modifiés. Les points représentés par des étoiles ont été obtenus par différence ΔU_{SG} entre deux mesures électroniques réalisées avant et après 4 dépôts locaux avec des concentrations différentes en ADN (Ref. = 0 µM, 5 µM 10 µM, 20 µM). Ils montrent la variation ΔU_{SG} de la tension U_{SG} qui est observée dans les caractéristiques des transistors et qui est due aux dépôts locaux d'ADN. Les carrés montrent l'intensité de la fluorescence mesurée sur les transistors FET séchés, une fois la mesure électronique réalisée avec l'électrolyte. On notera que la même détection électronique est obtenue avec des oligonucléotides du même type, mais non modifiés.

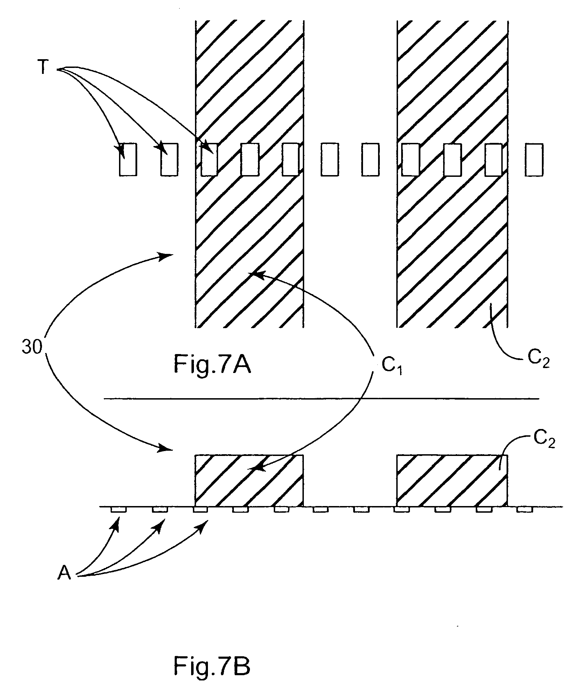

Les figures 7a et 7b montrent un circuit intégré présentant des transistors T arrangés selon une ligne (ou plusieurs lignes). Deux canaux microfluidiques (par exemple parallèles) C₁ et C₂ d'un substrat 30 permettent de mettre un ou plusieurs transistors T à effet de champ en contact avec une solution de référence ou une solution contenant des molécules cibles qui circule dans un canal C₁ et/ou C₂. Le matériau d'un substrat 30 qui comporte les canaux microfluidiques (ou capillaires) peut être un polymère PDMS (polydiméthylsiloxane) ou autre, un verre, du silicium, etc...

Il est ainsi possible de réaliser des mesures différentielles à partir de deux solutions qui circulent dans les deux canaux C₁ et C₂. Il est également possible de réaliser sur un même substrat 30 un grand nombre de tels micro-canaux fluidiques, le substrat dans lequel ils sont ménagés étant solidarisé au substrat semi-conducteur dans lequel sont intégrés les transistors à effet de champ FET. Il est également possible de mesurer une variation à l'intérieur d'un canal donné. Cette variation peut être au cours du temps. Il est également possible d'injecter différentes solutions dans un capillaire et le profil des concentrations reste inchangé le long du canal, même loin du point d'injection. On se reportera à l'article de Paul J. A. KENIS et al., intitulé "Microfabrication inside Capillaries Using Multiphase Laminar Flow Patterning" paru dans SCIENCE, vol. 285, 2 Juillet 1999, p. 83-85 (notamment figure 1a).

Une technique d'analyse mettant en oeuvre la microfluidique est décrite dans l'article "Monolithic integrated microfluidic DNA amplification and capillary electrophoresis analysis system" de Eric T. LAGALLY et collaborateurs paru dans Sensors and Actuators B 63 (2000) p. 138-146.

La détection d'au moins une interaction spécifique entre des biomolécules sondes et les biomolécules cibles est avantageusement réalisée en mettant en oeuvre un tampon de mesure dont la concentration en sel (par exemple KCI) est inférieure à celle du tampon de réaction.

Les biomolécules concernées (sources ou cibles) peuvent être par exemple de l'ADN, de l'ARN, des protéines et des vitamines.

Les interactions spécifiques peuvent être par exemple des interactions ADN-ADN, ADN-ARN, ADN-protéines, ARN-protéines, protéines-protéines, ou bien encore vitamines-protéines. L'ADN peur être l'ADN oligonucléotide synthétisé chimiquement. On peut également réaliser des interactions avec un acide peptide nucléique "PNA".

Dans les exemples suivants, des étapes supplémentaires de mesure ont été rajoutées afin de vérifier l'état de surface du capteur (transistors FET), la qualité de la fixation des biomolécules sondes et la reproductibilité des mesures.

### EXEMPLES

### Produits :

NaOH: 60 microlitres NaOH 16N, 420 microlitres d'éthanol, 220 microlitres d'eau.
PLL: solution de poly-L-lysine, P8920 (Sigma), 0,01% w/v dans un tampon PBS 0,1x.
   Oligonucléotide ARS3: 5' CCG CGA ACT GAC TCT CCG CC
   Oligonucléotide ARS5: 5' CAG GCG GCA GGG CTG ACG TT
   Oligonucléotide Cy3-ARS3sens: (complémentaire à ARS3 et avec fluorophore Cy3)
   Oligonucléotide Cy5-ARS5sens: (complémentaire à ARS5 et avec fluorophore Cy5)

### EXEMPLE 1 : Hybridation dans un tampon KCI de 50 mM et mesure avec un tampon KCl de 0,01 mM :

L'exemple montre qu'on peut obtenir des signaux plus importants en diminuant la concentration en sel de l'électrolyte de mesure par rapport à celle de l'hybridation. L'hybridation spécifique est vérifiée par une mesure de contrôle par fluorescence.

### 1. Traitement global de la surface SiO₂

Incubation 1 minute dans de l'acide sulfochromique, puis rinçage sous un courant d'eau déionisée, et séchage à l'air comprimé. Ce cycle d'incubation, rinçage, séchage est répété une fois.

Incubation 4 minutes dans la solution de NaOH.

Rinçage à l'eau et séchage.

### 2. Mesure électronique après traitement NaOH

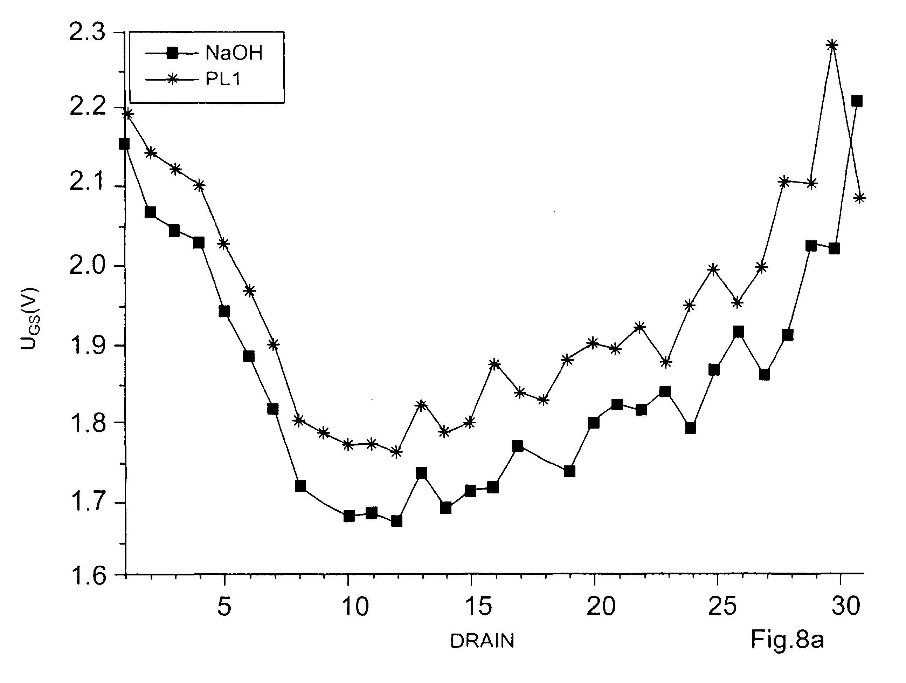

Tampon de mesure: KCI à la concentration de 0,01 mM. (voir figure 8a)

Cette mesure est suivie d'un rinçage à l'eau et d'un séchage.

### 3. Traitement global poly-L-lysine

On réalise une incubation pendant 2h.

Cette incubation est suivie d'un rinçage à l'eau et d'un séchage.

### 4. Mesure électronique "PL1".

Tampon de mesure: KCI 0,01 mM (voir figure 8a).

Cette mesure est suivie d'un rinçage à l'eau et d'un séchage.

### 5. Mesure électronique "PL2".

Tampon de mesure: KCI 0,01 mM.

Cette mesure est suivie d'un rinçage à l'eau et d'un séchage.

Cette deuxième mesure a pour but de vérifier la stabilité de la mesure à ce stade.

### 6. Dépôt des sondes d'ADN.

Avec une micropipette, on dépose sur la partie gauche du réseau de FET 0,2 microlitres d'une solution contenant l'oligonucléotide ars5. Sur la partie droite du réseau, on dépose 0,2 microlitres d'une solution contenant l'oligonucléotide Ars3. Dans les deux cas, les dilutions contiennent 1 micromole d'oligonucléotide dans un tampon KCI de 50mM. On réalise une incubation pendant 15 minutes, en atmosphère humide, pour éviter le séchage.

Cette incubation est suivie d'un rinçage à l'eau et d'un séchage.

### 7. Mesure électronique "Sonde1"

On utilise un tampon de mesure: KCI 0,01 mM.

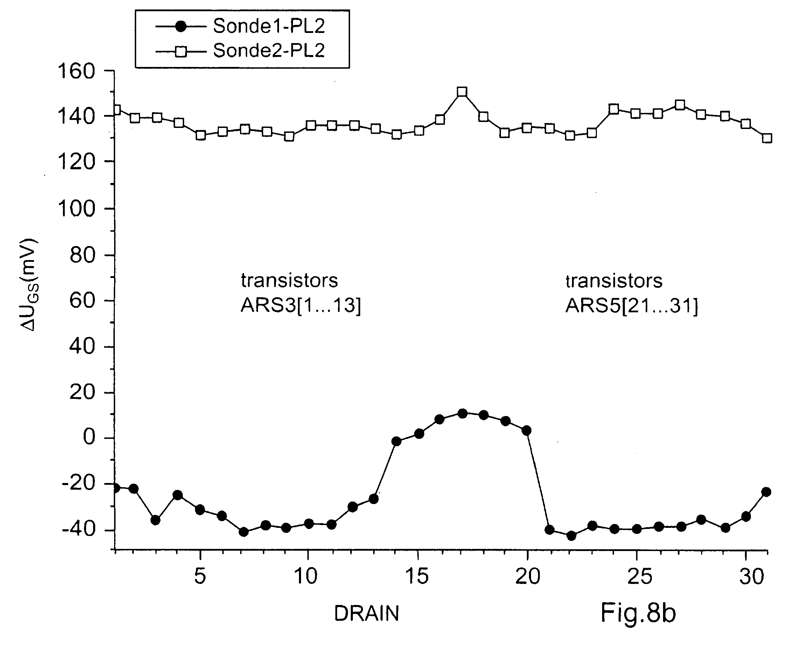

La Figure 8b montre les différences Delta U_{GS} entre la mesure "Sonde1" effectuée après le dépôt des biomolécules sondes et la mesure PL2 (étape 5) effectuée avant ce dépôt.

La courbe "Sonde1-PL2" (concentration en KCl : 0,01 Mm) montre un décalage de 25 mV pour les deux régions de dépôt gauche (dépôt de Ars3 sur les transistors 1 à 13) et droite (dépôt de Ars5 sur les transistors 21 à 31). Avec cette valeur, nous avons une concentration suffisante de biomolécules sondes et ne sommes pas encore trop proches de la saturation.

On réalise ensuite un pompage de l'électrolyte et on le remplace par 1 ml de KCI à 50 mM, sans séchage.

### 8. Mesure électronique "Sonde2"

Tampon de mesure: KCI à la concentration de 50 mM.

Comme le montre la Figure 8b, on ne voit plus les dépôts.

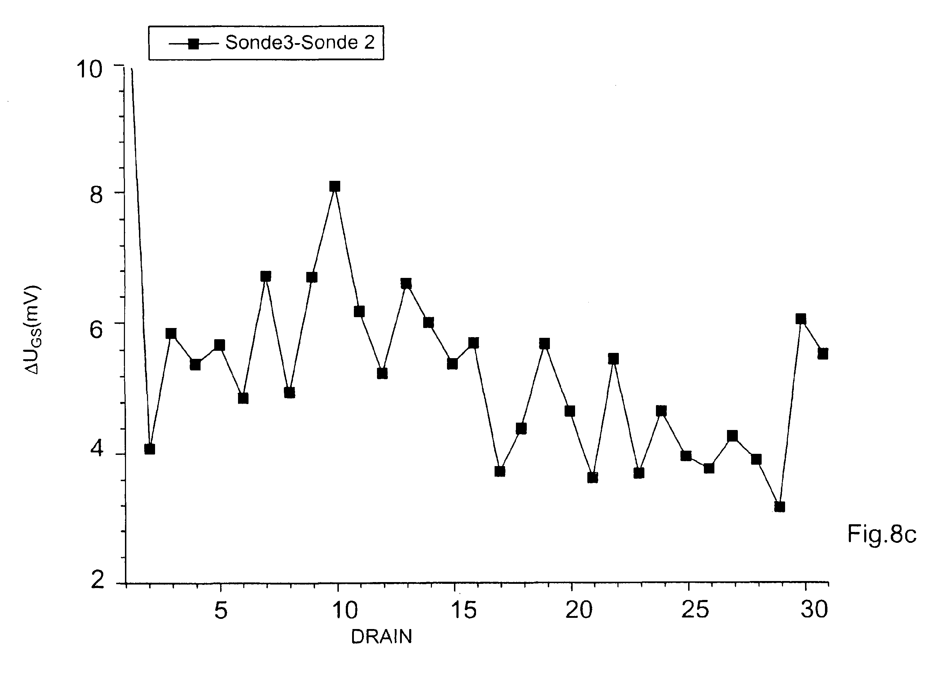

Pas de rinçage. Une mesure électronique "Sonde 3" est réalisée (KCI à 50 mM) pour vérifier la stabilité (Figure 8c).

### 9. Hybridation 1 (avec Cy5-Ars5sens).

Lors de la mesure "Sonde2" (étape 8) il y avait 1 ml de KCI sur les transistors FET.

Après, on ajoute directement (sans rinçage) 100 µl de Cy5Ars5sens à 1 µM.

La dilution finale en oligonucléotides est alors de l'ordre de 100 nM. On agite par pompage et on redépose 2 fois cette solution. Après agitation, la réaction de reconnaissance se fait pendant 5 minutes à l'abri de la lumière (pour éviter le blanchiment ou "bleaching" des fluorophores).

Après 5 minutes on rince: pompage de l'électrolyte et ajout de 1 ml de KCI

50mM, agitation, suivie d'un nouveau pompage ..., Ce cycle est réitéré 3 fois. Ces rinçages arrêtent la réaction. Pendant cette étape il n'y a à aucun moment séchage sur la surface.

### 10. Mesure électronique "Hyb1"

On plonge l'électrode dans le tampon de KCI 50 mM et fait la mesure électronique.

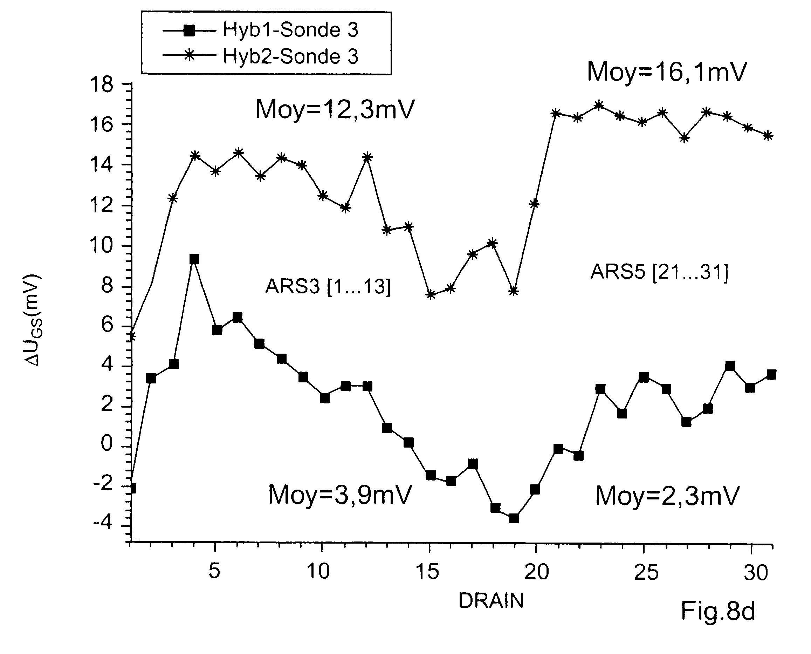

La courbe résultante Hyb1-Sonde 3 est représentée à la Figure 8d .

Le décalage Delta U_{SG} entre les mesures référencées Hyb1 et Sonde3 est d'environ +3.9 mV sur la région Ars3 et d'environ +2.3 mV sur la région Ars5 (ici les biomolécules sondes et les biomolécules cibles ont des séquences complémentaires). Ce décalage est d'environ -1.6 mV sur la région centrale sans dépôt de sondes d'ADN (transistors 14 à 20).

Après cette mesure on rince: pompage de l'électrolyte et ajout de 1 ml de KCI 0,01 mM, agitation, repompage ..., réiteration 5 fois. Pendant cette étape il n'y a à aucun moment séchage sur la surface.

### 11. Mesure électronique "Hyb12"

On plonge l'électrode de mesure dans le tampon de KCI 0,01 mM et on fait la mesure électronique.

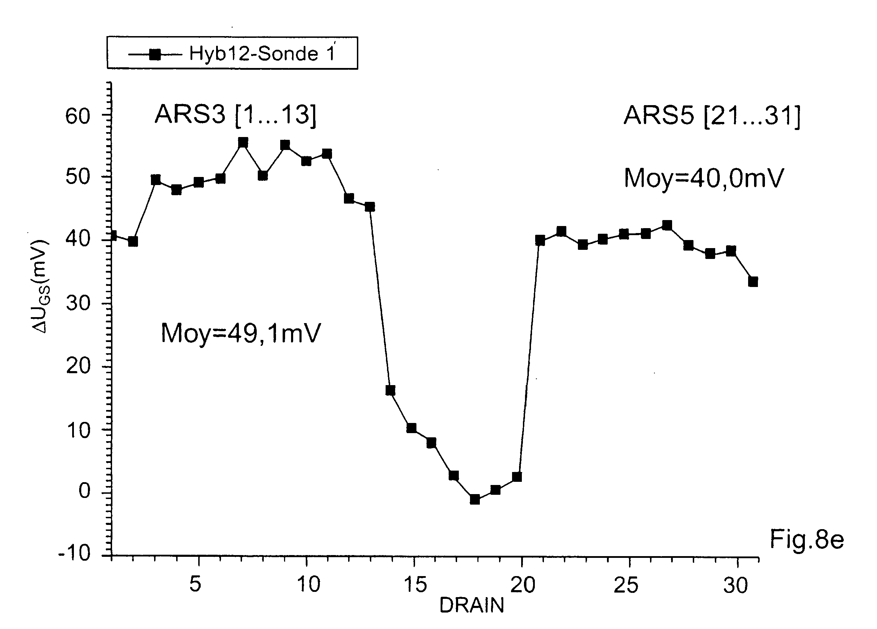

La courbe résultante (Hyb12 - Sonde 1) est représentée à la figure 8e. Les signes des décalages correspondent à ceux qui ont été observés à 50 mM (mesure "Hyb1"), mais les niveaux de ces décalages sont plus importants.

Dans la région Ars5 (Ars3) on observe un décalage moyen de +40 mV (+49 mV) entre les mesures "Hyb12" et "Sonde1", effectuées à 0,01 mM KCI.

### 12. Hybridation 2 (avec Cy3-Ars3sens)

On pompe l'électrolyte KCI 0,01 mM et on le remplace par 1 ml de KCI 50 mM. Après, on ajoute directement (sans rinçage) 100 µl de Cy3Ars3sens à 1 µM.

La dilution finale en oligonucléotides est alors de l'ordre de 100 nM.

On agite par pompage et on redépose 2 fois cette solution . Après agitation, la réaction de reconnaissance se fait pendant 5 minutes à l'abri de la lumière.

Une fois ces 5 minutes écoulées, on rince, puis on pompe l'électrolyte après quoi on ajoute 1 ml de KCI à 50mM. On agite, on pompe de nouveau. Ce cycle (rinçage, agitation, pompage) est réitéré 3 fois. Ces rinçages terminent la réaction. Pendant cette étape il n'y a à aucun moment séchage sur la surface.

### 13. Mesure électronique "Hyb2"

On plonge l'électrode dans le tampon de KCI à 50 mM et on effectue la mesure électronique. Le résultat de ces mesures est représenté par la courbe Hyb2 - Sonde3 de la Figure 8d.

On observe sur la figure 8d, que la différence Delta U_{SG} entre les mesures référencées

Hyb1 et Sonde3 est plus positive sur la région Ars3 (à gauche) que sur la région Ars5 (à droite, où les sondes et cibles ont des séquences complémentaires pour l'hybridation 1).Cette tendance est inversée pour la différence entre Hyb2 et Sonde2, en accord avec le fait que pour l'hybridation 2 les biomolécules sondes et cibles ont des séquences complémentaires sur la région Ars3.

Après cette mesure on rince: pompage de l'électrolyte et ajout de 1 ml de

KCI 0,01 mM, agitation, repompage ..., réiteration 5 fois.

Pendant cette étape il n'y a à aucun moment séchage sur la surface.

### 14. Mesure électronique "Hyb22".

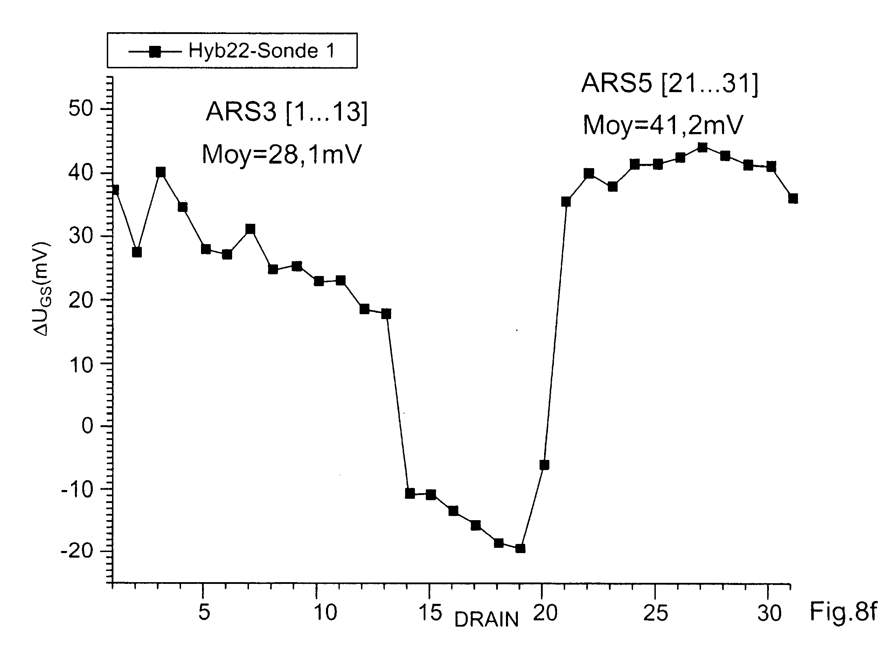

On plonge l'électrode dans le tampon de KCI 0,01 mM et fait la mesure électronique. Le résultat est représenté par la courbe Hyb22 - Sonde 1 de la Figure 8f.

Les signes des décalages sont renversés par rapport à ceux observés à 50 mM (mesure "Hyb2") et les signaux sont plus importants.

Dans la région Ars5 (Ars3) on observe un décalage moyen de -11 mV (+1 mV) entre les mesures "Hyb22" et "Hyb12".

Après cette mesure, on réalise un rinçage à l'eau et un séchage.

### 15. Mesures de fluorescence.

Avec un dispositif de mesure de fluorescence à deux couleurs on vérifie l'hybridation spécifique.

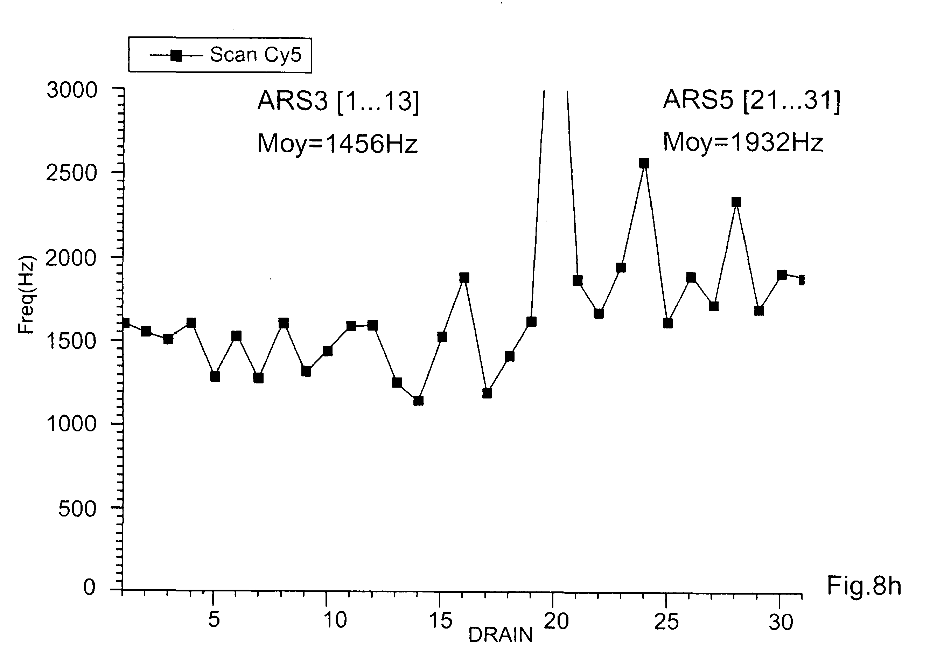

Sous excitation à 633 nm (Cy5) on observe plus de fluorescence sur la région Ars5 (Figure 8h).

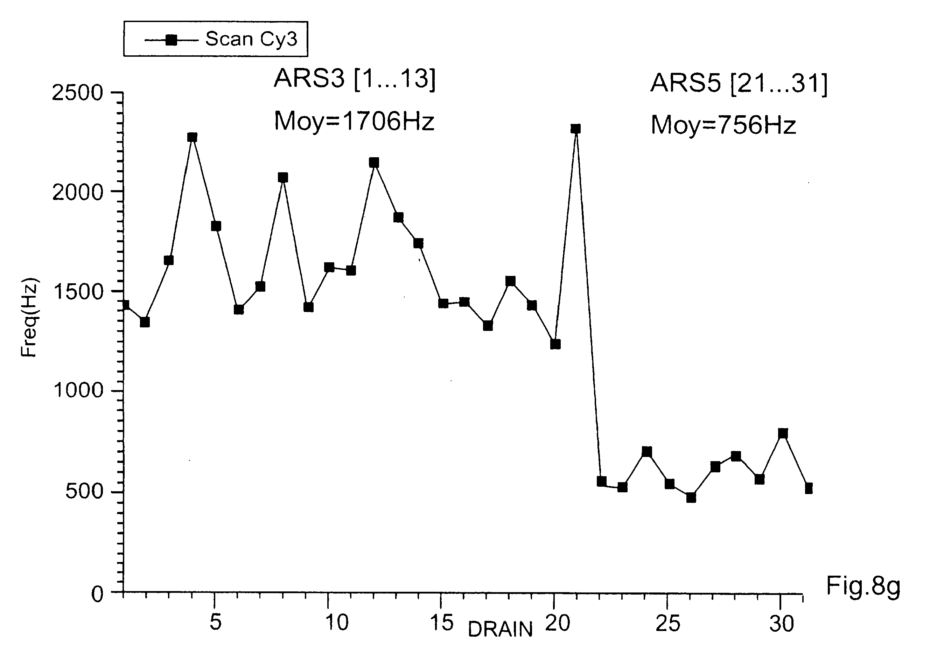

Sous excitation à 532 nm (Cy3) on observe plus de fluorescence sur la région Ars3 (Figure 8g).

### CONCLUSION

Mesures à 50 mM et hybridation avec Ars5Sens: le décalage de la région Ars5 est 1.6 mV plus négatif que celui de la région Ars3.

Mesures à 50 mM et hybridation avec Ars3Sens: le décalage de la région Ars3 est 5,4 mV plus négatif que celui de la région Ars5.

Mesures à 0,01 mM et hybridation avec Ars5Sens: le décalage de la region Ars5 est 9 mV plus négatif que celui de la région Ars3.

Mesures à 0,01 mM et hybridation avec Ars5Sens: le décalage de la region Ars3 est 22 mV plus négatif que celui de la région Ars3.

L'hybridation spécifique est vérifiée par la mesure de fluorescence.

### EXEMPLE 2 : Hybridation avec micro-dépôt et changement de tampon 20 mM/0.01 mM.

Les étapes 1 à 5 sont identiques à celles de l'exemple 1.

### 6- Dépôt des sondes d'ADN.

Avec un système de microdéposition commercial (GeSim, Rossendorf, Allemagne, Nano-plotter NP1), on dépose sensiblement 0,2 nl d'une solution contenant l'oligonucléotide ars5 sur la partie gauche du réseau de transistors FET. Sur la partie droite de ce réseau, on dépose 0,2 nl d'une solution contenant l'oligonucléotide Ars3. Au centre on dépose un mélange d' Ars5 et d' Ars3. Dans tous les cas, les dilutions contiennent 1µM (1 micromole) d'oligonucléotide dans un tampon d'eau déionisée. Les sondes sèchent quelques secondes après le dépôt à température et humidité ordinaires.

Pas de rinçage.

### 7. Mesure électronique "Sonde1"

Tampon de mesure: KCI 0,01 mM.

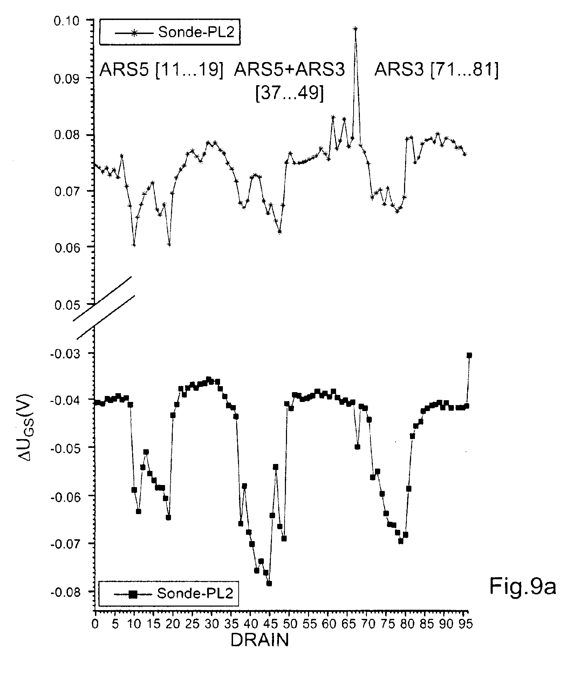

La courbe Sonde 1-PL2 de la Figure 9a montre les différences Delta U_{SG} entre des mesures faites après le dépôt des sondes et la mesure PL2 effectuée avant ce dépôt (avec le même tampon de mesure).

La courbe "Sonde1-PL2" montre que le dépôt des oligonucléotides a induit des décalages Delta U_{SG} compris entre -20 et -25 mV.

La mesure est suivie d'un pompage de l'électrolyte et son remplacement par 1 ml de KCI à 20 mM, sans séchage.

### 8. Mesure électronique "Sonde2"

Tampon de mesure: KCI 20 mM.

Les résultats sont représentés à la Figure 9a par la courbe Sonde 2-PL2 (tampon de mesure 20 mM). On voit toujours les dépôts, mais les pics sont plus petits qu'avec le tampon de mesure à 0,01 mM.

Pas de rinçage.

### 9. Mesures électroniques "Sonde3" et "Sonde4"

Tampon de mesure: KCI 20 mM.

Ces mesures ont pour but de vérifier la stabilité.

Pas de rinçage.

### 10. Hybridation 1 (avec Cy3-Ars5sens)

Lors de la mesure "Sonde4", il,y avait 1 ml de KCI sur les FETs. Après, on ajoute directement (sans rinçage) 100 µl de Cy3Ars5sens à 1 µM. La dilution finale en oligonucléotides est alors d'environ 100 nM. On agite par pompage et on redépose 2 fois cette solution. Après agitation, la réaction de reconnaissance se fait pendant 5 minutes à l'abri de la lumière.

Après ces 5 minutes on rince: pompage de l'électrolyte et ajout de 1 ml de KCl

20 mM, ce cycle étant réitéré 3 fois. Ces rinçages terminent la réaction. Pendant cette étape il n'y a à aucun moment séchage sur la surface.

### 11. Mesure électronique "Hyb1"

On plonge l'électrode dans le tampon de KCI 20 mM et on effectue la mesure électronique.

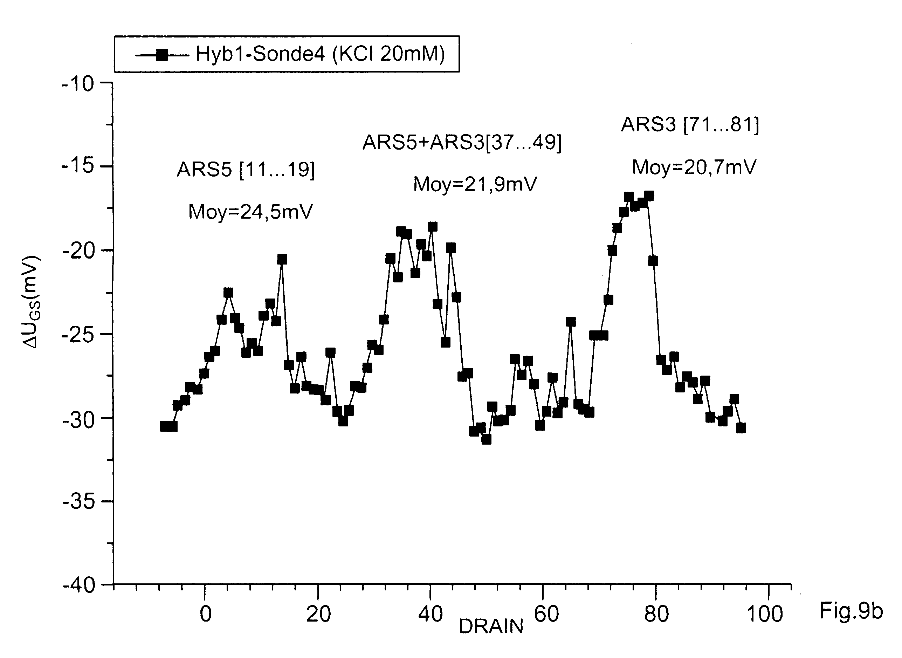

Le résultat est représenté par la courbe "Hyb1 - Sonde 4" de la Figure 9b.

Le décalage Delta U_{SG} entre les mesures référencées Hyb1 et Sonde 4 est d'environ -21 mV sur la région Ars3 (transistors 71 à 81) et d'environ -25 mV sur la région Ars5 (transistors 11 à 19). Ici, sur la région Ars5, les biomolécules sondes et cibles ont des séquences complémentaires.

Après cette mesure on rince: pompage de l'électrolyte et ajout de 1 ml de KCI 0.01mM, agitation, repompage ..., réiteration 5 fois.

Pendant cette étape il n'y a à aucun moment séchage sur la surface.

### 12. Mesure électronique "Hyb12"

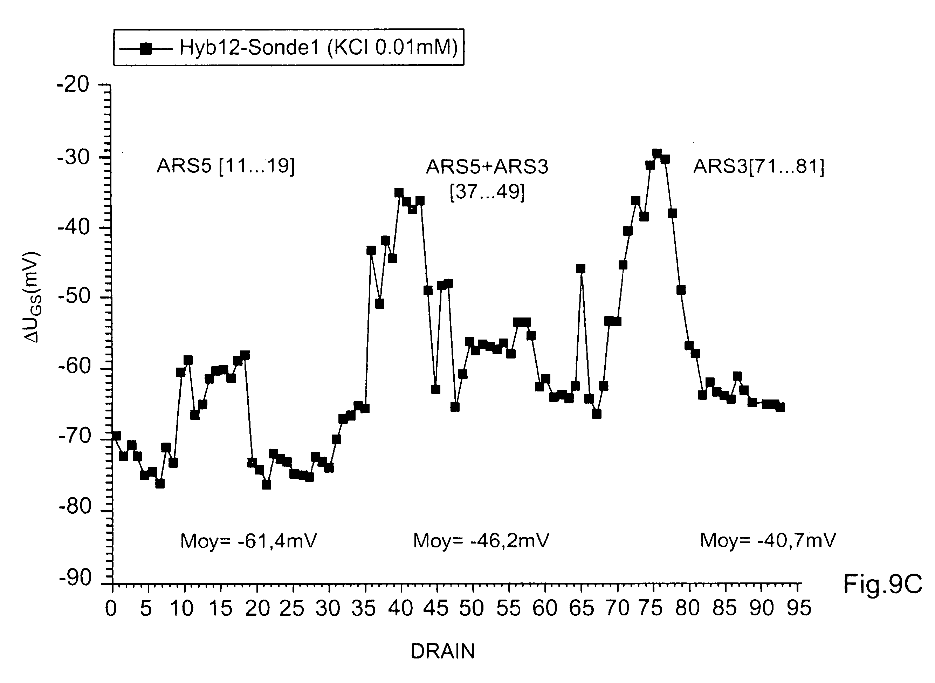

On plonge l'électrode dans le tampon de KCI 0,01 mM et on effectue la mesure électronique. Le résultat est représenté à la Figure 9c.

Les signes des décalages correspondent à ceux qui ont été observés avec un tampon de mesure de concentration 20 mM, mais les signaux sont plus importants.

Dans la région Ars5 (Ars3) on observe un décalage moyen de -61 mV (-41 mV) entre les mesures "Hyb12" et "Sonde1".

Après cette mesure on rince: pompage de l'électrolyte et ajout de 1 ml de

KCI 20 mM, agitation, repompage ..., réitération . 5 fois. Pendant cette étape il n'y a à aucun moment séchage sur la surface.

### 13. Mesure de fluorescence.

Avec un dispositif de mesure de fluorescence à deux couleurs on vérifie l'hybridation spécifique.

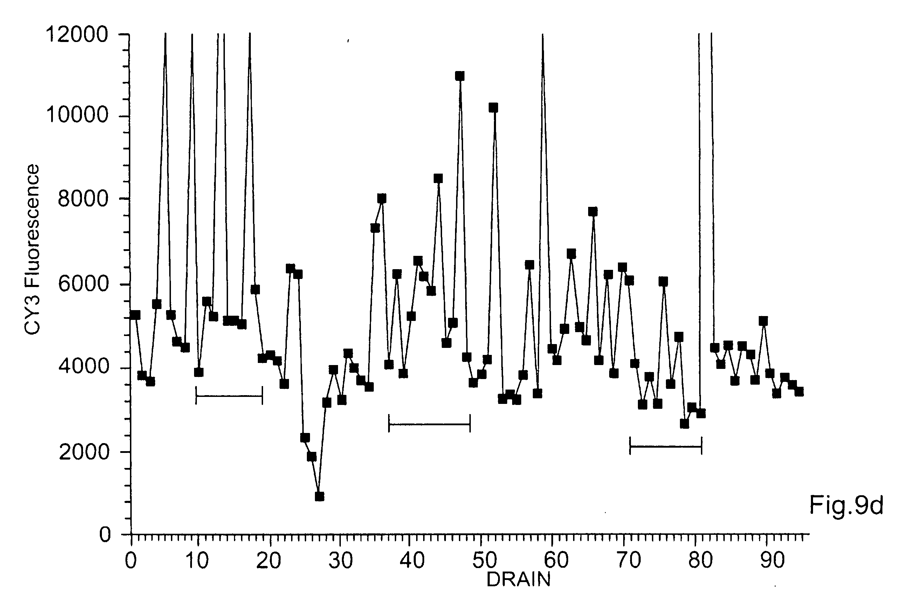

Sous excitation à 532 nm (figure 9d), on observe plus de fluorescence sur la région Ars5. Ceci confirme l'hybridation spécifique.

### CONCLUSIONS

Mesures à 20 mM et hybridation avec Ars5Sens: le décalage de la région Ars5 est 3,8 mV plus négatif que celui de la région Ars3.

Mesures à 0,01 mM et hybridation avec Ars5Sens: le décalage de la région Ars5 est 20,7 mV plus négatif que celui de la région Ars3.

L'hybridation spécifique est vérifiée par la mesure de fluorescence.

La mesure de référence réalisée avec des molécules sondes non soumises ou non encore soumises à une interaction avec des molécules cibles peut être effectuée sur une ou plusieurs zones actives distinctes de celle ou celles où intervient une dite réaction d'interaction avec des molécules cibles. Les zones actives d'un même circuit intégré peuvent être utilisées pour différentes interactions, ce qui permet d'effectuer en parallèles plusieurs mesures de types différents correspondant par exemple à différentes interactions, et/ou à l'étude des différentes biomolécules cibles. On peut ainsi détecter plusieurs interactions par rapport à une ou plusieurs mesures de référence réalisée sur des molécules sondes non soumises à interactions. On peut également détecter des interactions par comparaisons entre elles de mesures réalisés sur des molécules ayant été soumises à différentes interactions, par exemple entre des molécules sondes de même type, par exemple ADN, ayant ou non les mêmes séquences, et des biomolécules cibles identiques ou différentes.

Deux exemples d'applications potentielles de l'invention sont donnés ci-après :

Un exemple est l'étude de l'expression des gènes. On s'intéresse ici à la quantité relative des différentes molécules d'ARN messagers, extraites des cellules. On greffe des molécules sondes d'ADN qui portent des séquences de bases différentes à des endroits différents de la surface de la puce. Chaque sonde d'ADN est choisie de façon à interagir spécifiquement avec un type de molécule d'ARN (caractérisé par sa séquence). Le nombre relatif de molécules d'ARN hybridés sur deux régions de sondes différentes donne l'abondance relative de deux types de molécules d'ARN (et avec cela le niveau relatif de l'expression des deux gènes correspondants). L'intérêt est de suivre un grand nombre de gènes en parallèle, pour des études du développement, pour une caractérisation génétique des pathologies, pour une analyse moléculaire de l'effet des médicaments, etc...

Un autre exemple concerne la détection des mutations sur puces à ADN. Ici on cherche à analyser l'ADN d'un patient, en cherchant en parallèle la présence (ou l'absence) de plusieurs mutations connues. Pour cela, on greffe différents oligonucléotides (typiquement des 12mers) à différents endroits de la puce. Les séquences de base et les conditions d'hybridation (sel, température, etc) sont optimisées pour que la présence d'une mutation (même ponctuelle) induise une différence mesurable dans les taux d'hybridation des différentes sondes. L'échantillon de molécule cible consiste ici souvent en des molécules d'ADN double brin obtenues par "PCR" à partir d'une faible quantité d'ADN génomique du patient.

Dans les deux cas, on s'intéresse à la différence des signaux induits par l'hybridation d'un échantillon complexe contenant un grand nombre de biomolécules cibles différentes sur une puce avec plusieurs régions de sondes différentes (souvent le même type de molécule mais avec une séquence différente). Cette configuration est très intéressante pour la détection électronique des interactions.

On comprend que les expressions « deux interactions différentes » et « autre interaction » couvrent la situation où cette différence d'interaction correspond à deux sondes de type ou de séquence différente, mais en contact avec une même solution de molécule cible.

La détection d'une interactivité spécifique peut s'effectuer sur une ou plusieurs régions de porte du réseau de transistors à effet du champ FET. L'avantage d'utiliser plusieurs régions de porte permet de mettre en évidence les inhomogénéités.

Alternativement, l'utilisation d'un seul transistor du réseau est possible pour des biomolécules sondes d'un type donné et une interaction donnée. Si on utilise un transistor de grandes dimensions, on obtient directement une mesure moyennée des inhomogénéités.

## Revendications

1. Procédé de détection électronique d'au moins une interaction spécifique entre des molécules sondes fixées sur au moins à une zone active d'un capteur et des biomolécules cibles, **caractérisé en ce que** ledit capteur est constitué par un réseau de transistors à effet de champ (T₁, T₂, ...) dont chacun présente une région de source (5), une région de drain (D), ainsi qu'une région de porte qui constitue une zone active (3) sur laquelle ladite interaction spécifique doit être détectée, et **caractérisé en ce qu'**il comporte les étapes suivantes :
**a) mettre en contact des zones actives distinctes (3) avec des molécules sondes d'un type donné pour les fixer sur lesdites zones actives ;**
b) mettre en contact au moins certaines des molécules sondes avec des biomolécules cibles susceptibles d'interagir avec lesdites molécules sondes et effectuer une dite interaction spécifique dans un tampon réactionnel ayant une première concentration en sel ;
c) mesurer au moins un point de la caractéristique courant de drain/tension source-porte/tension source-drain d'au moins un transistor dudit réseau pour détecter ladite interaction spécifique au moins pour un point de mesure obtenu dans un tampon de mesure ayant une deuxième concentration en sel qui est inférieure à la première concentration pour des molécules sondes ayant été soumises à ladite interaction spécifique ;
ladite mesure étant effectuée de manière spatiale, soit par différence entre ledit point de mesure et un point de référence, dans un dit tampon de mesure, pour des molécules sondes fixées sur des zones actives (3) distinctes, le point de mesure étant obtenu pour des molécules sondes ayant été soumises à l'interaction de l'étape b) et le point de référence étant obtenu pour des molécules sondes n'y ayant pas été soumises, soit par différence entre deux points de mesure obtenus dans un dit tampon de mesure pour des molécules sondes fixées sur des zones actives (3) distinctes et ayant été soumises à deux interactions spécifiques différentes.

2. Procédé selon la revendication 1, **caractérisé en ce que** ledit point de référence est déterminé à partir de molécules sondes de même type que celles qui ont été soumises à ladite interaction spécifique, et présentant soit la même séquence, soit une séquence différente.

3. Procédé selon la revendication 1, **caractérisé en ce que** les molécules sondes soumises auxdites deux interactions spécifiques différentes sont du même type, qu'elles aient ou non des séquences identiques.

4. Procédé selon une des revendications précédentes, **caractérisé en ce que** ladite mesure d'au moins un point de la caractéristique met en oeuvre l'application d'une tension donnée (U_{DS}) entre le drain et la source d'au moins un transistor, ainsi que l'application dans un premier cas d'une tension donnée (U_{GS}) entre la porte et la source dudit transistor, dans un deuxième cas d'un courant de drain (I_{D}) donné audit transistor.

5. Procédé selon la revendication 4, **caractérisé en ce que** dans le premier cas, le point est obtenu en mesurant le courant de drain I_{D} et dans le deuxième cas en mesurant la tension U_{GS} entre la porte et la source.

6. Procédé selon une des revendications précédentes, **caractérisé en ce que** le tampon de mesure est du KCI.

7. Procédé selon une des revendications précédentes, **caractérisé en ce que** la concentration du tampon réactionnel est comprise entre 20m M et 1 M.

8. Procédé selon une des revendications 7, **caractérisé en ce que** la concentration du tampon de mesure est supérieur à 0,002m M et inférieur à 20m M.

9. Procédé selon la revendication 8, **caractérisé en ce que** la concentration du tampon de mesure est au moins égale à 0,01 m M.

10. Procédé selon une des revendications 8 ou 9, **caractérisé en ce que** la concentration du tampon de mesure est au plus égale à 15m M.

11. Procédé selon l'une des revendications précédentes, **caractérisé en ce que** le passage à un tampon de concentration inférieure est séparé par une étape de rinçage.

12. Procédé selon l'une des revendications précédentes, **caractérisé en ce que** les molécules sondes sont des molécules, notamment des biomolécules, susceptibles d'être reconnues par un type de biomolécule cible.

13. Procédé selon la revendication 12, **caractérisé en ce que** les molécules sondes et/ou les biomolécules cibles sont des molécules d'ADN, d'ARN ou de protéines, ou bien encore des vitamines.

14. Procédé selon la revendication 13, **caractérisé en ce que** les biomolécules sondes sont des molécules d'ADN et **en ce que** les transistors à effet de champ sont de type à canal n à appauvrissement, avec une polarisation de porte négative.

15. Procédé selon une des revendications précédentes, **caractérisé en ce que** qu'il comporte avant a) au moins un étape de mesure de contrôle avec un dit tampon de mesure.

16. Procédé selon une des revendications précédentes, **caractérisé en ce qu'**il comporte la circulation à travers au moins un microcanal fluidique d'au moins une solution qui constitue une référence ou qui contient des molécules cibles pour la mettre en contact avec au moins un dit transistor à effet de champ.

## Claims

1. Method for electronic detection of at least one specific interaction between probe molecules fixed to at least one active area of a sensor and target biomolecules, **characterized in that** said sensor consists of an array of field-effect transistors (T₁, T₂, ...) each of which has a source region (5), a drain region (D) and a gate region that constitutes an active area (3) on which said specific interaction must be detected and **characterized in that** it includes the following steps:
a) bringing the different active areas (3) into contact with probe molecules of a given type to fix them to said active areas;
b) bringing at least some of the probe molecules into contact with target biomolecules liable to interact with said probe molecules and to undergo one of said specific interactions in a reaction buffer having a first salt concentration;
c) measuring at least one point on the drain current/ source-gate voltage/ source-drain voltage characteristic of at least one transistor of said array to detect said specific interaction at least for a measurement point obtained in a measurement buffer having a second salt concentration that is lower than the first concentration for probe molecules that have been subjected to said specific interaction;
said measurement being effected spatially, either as the difference between said measurement point and a reference point in one of said measurement buffers for probe molecules fixed to different active areas (3), the measurement point being obtained for probe molecules that have been subjected to the interaction of step b) and the reference point being obtained for probe molecules that have not been subjected thereto, or as the difference between two measurement points obtained in one of said measurement buffers for probe molecules fixed to different active areas (3) that have been subjected to two different specific interactions.

2. Method according to claim 1 **characterized in that** said reference point is determined from probe molecules of the same type as those that have been subjected to said specific interaction and have either the same sequence or a different sequence.

3. Method according to claim 1 **characterized in that** the probe molecules subjected to said two different specific interactions are of the same type, whether they have identical sequences or not.

4. Method according to any one of the preceding claims **characterized in that** said measuring of at least one point on the characteristic entails applying a given voltage (U_{DS}) between the drain and the source of at least one transistor and in a first case applying a given voltage (U_{GS}) between the gate and the source of said transistor and in a second case applying a given drain current (I_{D}) to said transistor.

5. Method according to claim 4 **characterized in that** the point is obtained in the first case by measuring the drain current I_{D} and in the second case by measuring the voltage U_{GS} between the gate and the source.

6. Method according to any one of the preceding claims **characterized in that** the measurement buffer is KCl.

7. Method according to any one of the preceding claims **characterized in that** the concentration of the reaction buffer is in the range 20 mM to 1 M.

8. Method according to claim 7 **characterized in that** the concentration of the measurement buffer is greater than 0.002 mM and less than 20 mM.

9. Method according to claim 8 **characterized in that** the concentration of the measurement buffer is equal to at least 0.01 mM.

10. Method according to either of claims 8 or 9 **characterized in that** the concentration of the measurement buffer is equal to at most 15 mM.

11. Method according to any one of the preceding claims **characterized in that** changing to a buffer of lower concentration is preceded by a rinsing step.

12. Method according to any one of the preceding claims **characterized in that** the probe molecules are molecules, in particular biomolecules, that can be recognised by a target biomolecule type.

13. Method according to claim 12 **characterized in that** the probe molecules and/or the target biomolecules are molecules of DNA, RNA, proteins or vitamins.

14. Method according to claim 13 **characterized in that** the probe biomolecules are molecules of DNA and the field-effect transistors are of the depleted n-channel type with negative gate bias.

15. Method according to any one of the preceding claims **characterized in that** it includes before the step a) at least one control measurement step using said measurement buffer.

16. Method according to any one of the preceding claims **characterized in that** it includes circulating at least one solution that constitutes a reference or that contains target molecules through at least one fluidic microchannel to bring it into contact with at least one of said field-effect transistors.

## Patentansprüche

1. Verfahren zur elektronischen Detektion zumindest einer spezifischen Wechselwirkung zwischen Sonden-Molekülen, die an zumindest einem Wirkbereich eines Sensors fixiert sind, und Ziel-Biomolekülen, **dadurch gekennzeichnet, dass** der Sensor aus einem Netz von Feldeffekttransistoren (T₁, T₂, ...) besteht, von denen jeder einen Source-Bereich (5), einen Drain-Bereich (D) sowie einen Gate-Bereich aufweist, der einen Wirkbereich (3) bildet, in dem die spezifische Wechselwirkung detektiert werden muss, und **dadurch gekennzeichnet, dass** es die nachfolgenden Schritte umfasst:
a) In-Kontakt-Bringen der verschiedenen Wirkbereiche (3) mit Sonden-Molekülen eines gegebenen Typs, um sie an den Wirkbereichen zu fixieren,
b) In-Kontakt-Bringen zumindest bestimmter der Sonden-Moleküle mit den Ziel-Biomolekülen, welche geeignet sind, mit den Sonden-Molekülen zu interagieren, und Erzielen einer spezifischen Wechselwirkung in einem Reaktionspuffer mit einer ersten Salzkonzentration;
c) Messen zumindest eines Punktes der Kenngröße Drain-Strom/Source-Gate-Spannung/Source-Drain-Spannung von zumindest einem Transistor des Netzes, um die spezifische Wechselwirkung zumindest für einen erhaltenen Messpunkt in einem Messpuffer mit einer zweiten Salzkonzentration zu detektieren, die geringer als die erste Konzentration bei Sonden-Molekülen ist, die der spezifischen Wechselwirkung ausgesetzt wurden;
wobei die Messung räumlich entweder durch die Differenz zwischen dem Messpunkt und einem Referenzpunkt in einem sogenannten Messpuffer bei Sonden-Molekülen erfolgt, die an den verschiedenen Wirkbereichen (3) fixiert sind, wobei der Messpunkt bei Sonden-Molekülen erhalten wird, die der Wechselwirkung aus Schritt b) ausgesetzt wurden, und wobei der Referenzpunkt bei Sonden-Molekülen erhalten wird, die dieser Wirkung nicht ausgesetzt wurden, oder aber durch die Differenz zwischen zwei Messpunkten, die in einem Messpuffer bei Sonden-Molekülen erhalten wurden, die an verschiedenen Wirkbereichen (3) befestigt sind und zwei unterschiedlichen spezifischen Wechselwirkungen ausgesetzt wurden.

2. Verfahren nach Anspruch 1, **dadurch gekennzeichnet, dass** der Referenzpunkt ausgehend von Sonden-Molekülen ermittelt wird, die vom gleichen Typ wie diejenigen sind, die der spezifischen Wechselwirkung ausgesetzt wurden, und entweder die gleiche Sequenz oder eine unterschiedliche Sequenz aufweisen.

3. Verfahren nach Anspruch 1, **dadurch gekennzeichnet, dass** die Sonden-Moleküle, die den beiden unterschiedlichen spezifischen Wechselwirkungen ausgesetzt werden, vom gleichen Typ sind, unabhängig davon, ob sie identische Sequenzen haben oder nicht.

4. Verfahren nach einem der vorangehenden Ansprüche, **dadurch gekennzeichnet, dass** die Messung von zumindest einem Punkt der Kenngröße das Anlegen einer gegebenen Spannung (U_{DS}) zwischen Drain und Source von zumindest einem Transistor sowie das Anlegen einer gegebenen Spannung (U_{GS}) zwischen Gate und Source des Transistors in einem Fall und das Anlegen eines gegebenen Drain-Stroms (I_{D}) an den Transistor in einem zweiten Fall umfasst.

5. Verfahren nach Anspruch 4, **dadurch gekennzeichnet, dass** in dem ersten Fall der Punkt durch Messung des Drain-Stroms I_{D} und in dem zweiten Fall durch Messung der Spannung U_{GS} zwischen Gate und Source erhalten wird.

6. Verfahren nach einem der vorangehenden Ansprüche, **dadurch gekennzeichnet, dass** der Messpuffer KCI ist.

7. Verfahren nach einem der vorangehenden Ansprüche, **dadurch gekennzeichnet, dass** die Konzentration des Reaktionspuffers zwischen 20 mM und 1 M beträgt.

8. Verfahren nach Anspruch 7, **dadurch gekennzeichnet, dass** die Konzentration des Messpuffers über 0,002 mM und unter 20 mM beträgt.

9. Verfahren nach Anspruch 8, **dadurch gekennzeichnet, dass** die Konzentration des Messpuffers zumindest gleich 0,01 mM ist.

10. Verfahren nach einem der Ansprüche 8 oder 9, **dadurch gekennzeichnet, dass** die Konzentration des Messpuffers höchstens gleich 15 mM ist.

11. Verfahren nach einem der vorangehenden Ansprüche, **dadurch gekennzeichnet, dass** der Übergang zu einem Puffer mit geringerer Konzentration durch einen Schritt des Spülens abgetrennt ist.

12. Verfahren nach einem der vorangehenden Ansprüche, **dadurch gekennzeichnet, dass** die Sonden-Moleküle Moleküle, insbesondere Biomoleküle, sind, die dazu geeignet sind, von einem Typ von Ziel-Biomolekül erkannt zu werden.

13. Verfahren nach Anspruch 12, **dadurch gekennzeichnet, dass** die Sonden-Moleküle und/oder Ziel-Biomoleküle DNA-, RNA- oder Protein-Moleküle oder auch Vitamine sind.

14. Verfahren nach Anspruch 13, **dadurch gekennzeichnet, dass** die Sonden-Biomoleküle DNA-Moleküle sind und dass die Feldeffekttransistoren vom n-Kanal-Verarmungstyp mit einer negativen Gate-Polarisierung sind.

15. Verfahren nach einem der vorangehenden Ansprüche, **dadurch gekennzeichnet, dass** es vor a) zumindest einen Kontrollmessschritt mit einem Messpuffer umfasst.

16. Verfahren nach einem der vorangehenden Ansprüche, **dadurch gekennzeichnet, dass** es das Durchströmen zumindest eines fluidischen Mikrokanals von zumindest einer Lösung umfasst, die eine Referenz darstellt bzw. Ziel-Moleküle enthält, um sie mit zumindest einem Feldeffekttransistor in Kontakt zu bringen.
